# EUROPEAN PATENT SPECIFICATION

(11) **EP 2 433 502 B1**
(45) Date of publication and mention of the grant of the patent: **04.05.2016**
(21) Application number: 10777800.3
(22) Date of filing: 20.05.2010
(51) Int. Cl.: A23L 27/00, A23L 2/56, A23L 2/70, A61K 47/46

(54) **BITTERNESS-SUPPRESSING AGENT**
BITTERKEITSUNTERDRÜCKUNGSMITTEL
AGENT DE SUPPRESSION D'AMERTUME

(30) Priority: 20.05.2009 JP 2009121833
(43) Date of publication of application: 28.03.2012
(73) Proprietor: Kirin Holdings Kabushiki Kaisha, Tokyo 104-8288 (JP)
(72) Inventor: MANABE, Fumitoshi, Kanagawa-ken (JP); KATAYAMA, Mikio, Kanagawa-ken (JP); IMASATO Youji, Tokyo 104-8288 (JP)
(74) Representative: Hoffmann Eitle
(86) International application number: PCT/JP2010/058507
(87) International publication number: WO 2010/134568

(56) References cited:
- WO-A1-2009/017116
- WO-A1-2010/026946
- JP-A- 61 111 673
- JP-A- H06 503 480
- JP-A- 2001 220 594
- JP-A- 2002 095 450
- JP-A- 2005 179 279
- JP-A- 2009 055 874
- US-A- 5 510 125

## Description

### TECHNICAL FIELD

The present invention relates to a bitterness suppressant derived from a juice of a stone fruit, and more specifically to a bitterness suppressant characterized by being derived from a juice of a stone fruit wherein a low molecular weight saccharide has been removed.

### BACKGROUND OF THE INTENTION

In recent years, many health-conscious food or beverage products have been developed and also commercially available. To produce a real product that provides a real feeling of health function, it is obvious that a preparation exceeding an effective amount is needed. As in an old saying: "a good medicine tastes bitter", a so-called functional component that has useful effect on human body often has bitterness. In that case, adding more than an effective amount of the functional component to a food or beverage product causes the reduction of taste preference, leading to the reduced attractiveness of the product.

For example, hop as an origin of a bitterness component contained in beer has been traditionally used also as a folk medicine and known to have various health functions, such as sedative effect and stomachic effect. If more than a certain amount of an extraction liquid obtained from the hop is added to a food or beverage product, a uniquely strong bitterness occurs, which can damage the taste preference of the product.

Other than that, as functional materials having bitterness, there are known components extracted from plants, such as catechin and tannin contained in large quantities in tea and wine, caffeine and chlorogenic acid contained in coffee in large quantities, and naringin and hesperidin contained in citrus fruit juice in large quantities, proteins such as casein and whey and peptide materials as degradation-products thereof, amino acids, various vitamins such as vitamin B1, trace minerals such as calcium, and the like.

Additionally, in pharmaceutical products also, many components have bitterness. Many of the pharmaceutical products are hard to take, which include basic drugs such as quinine and promethazine, Chinese medicines such as coptis root and Japanese green gentian, and crude medicines.

To remove or suppress such bitterness, many attempts have been reported. Examples of substances used as bitterness reduction materials include phosphatidic acid (product name: "BENECORT BMI" Kao Corp.) and L-ornithine (Food Science, No. 317, p54, 2004). However, any of these products do not always have strong effect when used alone, and particularly it has been difficult for them to suppress the bitterness of the above hop extract. In addition, in a masking technique by the addition of sweeteners such as sucralose and thaumatin (Japanese Patent Laid open publication No. 2008-99682), bitterness is more or less masked by sweetness, but the use of the technique is limited because of their strong sweetness.

Meanwhile, a bitter substance-inclusion-technique using cyclodextrin (Japanese Patent Laid open publication No. 2004-57153) has masking effect against a specific bitterness component but the effect is significantly Influenced by the degree of the inclusion.

In the case where the pharmaceutical product is a tablet, sugarcoating is usually mainly performed, and besides that, a film coating technique, micro-encapsulation or the like is used. However, complete masking of bitterness has been difficult. Furthermore, in the case of a liquid drug, like beverages, those techniques cannot be used. Thus, in the fields of food or beverage products and pharmaceutical products, bitterness suppression has still been a major problem.
JP2002-095450 (A) aims to provide a beverage containing apple polyphenol, mildened is astringency derived from the polyphenol. The document provides a beverage that is obtained by adding 3-30 wt.% juice to a beverage containing polyphenol corresponding to one apple per 500 ml beverage so as to milden astringency derived from the polyphenol. The juice may be grapefruit juice, mandarin orange juice, pineapple juice, lime juice, orange juice, grape juice, peach juice, pear juice, kiwi fruit juice, acerola juice, melon juice, strawberry juice, mango juice, passion fruit juice, muscat juice, banana juice, hawthorn juice, aloe juice, lemon juice, blueberry juice or juice products of their mixtures, and citric acid-added apple juice may be used.

### SUMMARY OF THE INTENTION PROBLEM TO BE SOLVED BY THE INVENTION

It is an object of the present invention to provide a means for suppressing the bitterness of food or beverage products and pharmaceutical materials having bitterness.

### MEANS FOR SOLVING THE PROBLEM

The present inventors have found strong bitterness suppression effect in a juice of a specific fruit, particularly in a juice of a stone fruit, and also have found that the same effect is exhibited by a stone fruit juice wherein a low molecular weight saccharide has been removed, whereby we have completed the invention.

Specifically, according to the present invention, there is provided a bitterness suppressant consisting of a juice of a stone fruit wherein a low molecular weight saccharide has been removed. The present invention provides a use of such a juice as defined in claim 4.

According to the present invention, there is also provided a method for producing a food or beverage product as defined in claim 3 whose bitterness is suppressed, the method comprising adding, to a food or beverage product having bitterness, a juice of a stone fruit wherein a low molecular weight saccharide has been removed. The present invention also relates to the food or beverage as defined in claim 1.

According to the present invention, there is also provided a method for producing a pharmaceutical product as defined in claim 3 whose bitterness is masked, the method comprising adding to a pharmaceutical product a juice of a stone fruit wherein a low molecular weight saccharide has been removed.
The present invention also relates to the pharmaceutical product as defined in claim 1.

The present invention allows for suppressing or masking of the bitterness of a food or beverage product and a pharmaceutical product having bitterness to provide a food or beverage product and a pharmaceutical product that can be easily taken. Particularly, the present invention using naturalfruit juice can respond to the natural food material orientation of consumers. Furthermore, from the juice used in the present invention, a low molecular weight saccharide is removed. Therefore, the juice is less sweet and has low calories, so that it can be widely used for various food or beverage products and pharmaceutical products.

### BRIER DESCRIPTION OF THE DRAWINGS

Fig. 1 shows results of evaluation example 1.
Fig. 2 shows results of evaluation example 2.
Fig. 3 shows results of evaluation example 3.

### DETAILED DESCRIPTION OF THE INTENTION

### [Stone Fruit-Derived Juice]

The present invention uses a juice of a stone fruit as an active ingredient for bitterness suppression.

Herein, the "stone fruit" means a kind of deciduous fruit tree, which is a fruit characterized by having one hard core in the center of the fruit. Examples of stone fruits include those belonging to the genus Prunus of the rose family, such as peach, nectarine, plum (prune), apricot, Japanese apricot, and cherry.

A stone fruit-derived juice used in the present invention may be obtained from any of the above-mentioned stone fruits. Preferred stone fruits are peach, apricot, and plum from the viewpoint of versatility, flavor, and hue of the fruits.

The method for juicing the fruit is not specifically limited and juicing can be done by an ordinal method. Specifically, juicing can be done by splitting the fruit as a raw material into half, peeling the skin of the fruit, removing a core, heating the fruit flesh for softening, then sieving through a pulper or finisher, and the like (for example, see "Science of Fruits" published by Asakura Publishing Co., Ltd.). Additionally, the form of fruit juice to be used is also not specifically limited. The fruit juice can be in any form, such as a straight juice, concentrated juice, clear juice, or turbid juice.

In the case where a puree or the like is used, it has a high solid content. Thus it is difficult to add the puree or the like as it is into a food product. Accordingly, the puree or the like may be used by degrading a high polymer to some extent by a polysaccharide-degrading enzyme (such as pectinase, cellulase, or hemicellulase) or the like used for ordinal clarification of juice and removing the solid content through filtering or the like. A by-product such as a vegetative residue obtained after juicing of the fruit can also be used like juice by removing a solid content by enzyme treatment and solid-liquid separation.

### [Method for Removing Low Molecular Weight Saccharides]

Low molecular weight saccharides contained in fruit juice are primarily observed to have bitterness suppression effect by sweetness. However, as shown in Examples, it was found that, even when a low molecular weight saccharide was removed from the fruit juice, a component other than that had bitterness suppression effect.

General sugar compositions of major stone fruits are as follows: peach (sucrose: 6 to 7%; fructose: 1%; glucose: 1%; and sorbitol: 0.3%), apricot (sucrose: 3.4%; fructose: 1.2%; glucose: 1.6%; and sorbitol: 0.4%), plum (sucrose: 4.3%; fructose: 2%; glucose: 5.1%; and sorbitol: 0.3%), and Japanese apricot (sucrose: 0%; fructose: 0.1%; glucose: 0.17%; and sorbitol: 0.3%). It is sucrose that controls the sweetness of fruit, and as the fruit becomes matured, a ratio of sucrose increases.

In the present invention, the low molecular weight saccharide removed from the fruit juice refers to any of sugars whose degrees of polymerization are 1 to 20, and specific examples of such sugars include monosaccharides, disaccharides, and oligosaccharides having degrees of polymerization of approximately 3 to 20, (such as glucose, sucrose, and fructose).

The method for removing a low molecular weight saccharide from a juice can be any method as long as it is a method that can remove a low molecule, and examples of the method include cellulose membrane dialysis, ultrafiltration, gel filtration chromatography, electrodialysis, alcohol precipitation, and enzyme treatment. Preferred is ultrafiltration that can efficiently remove a low molecule and can retain a high polymer fraction to concentrate the fraction as it is. The material of an ultrafiltration membrane may be polysulfone, polyacrylonitrile, or the like. A fractional molecular weight of a dialysis membrane, an ultrafiltration membrane or the like is preferably 3,000 to 50,000, and more preferably 4,000 to 10,000 from the viewpoints of removing low molecular weight saccharides, a relationship between film occlusion and removal time, and a yield of a high polymer fraction obtained.

The removal ratio of a low molecular weight saccharide in the present invention can be obtained by measurement of Brix using a saccharimeter. The removal ratio of a low molecular weight saccharide can be any as long as the ratio is in a range in which no sweetness is felt when added to a bitterness component. The removal ratio thereof can be preferably no less than 70% of a sugar content of the juice as a raw material, and more preferably no less than 90% thereof.

### [Bitterness Component]

Food or beverage products and pharmaceutical products to which the present invention is applicable can be either of those having originally a bitterness component or those having obtained bitterness by addition of a bitterness component.

The bitterness component is selected from humulones such as humulone and isohumulone contained in hop, or quinine.

Among bitterness components to which the present invention can be applied, preferred examples include isohumulone contained in isomerized hop extracts and quinine used as a pharmaceutical product.

An isomerized hop extract is obtained by isomerization of a product extracted from hop cone using a CO₂ supercritical extraction process. For more convenience, any of commercially available isomerized hop extracts can also be used.

Additionally, the present invention can be applied to tetrahydroisohumulone and p-isohumulone that are produced by oxidation-reduction reaction to be used as a substitute or complementary product of hop.

Examples of quinines to be used include quinine hydrochloride and quinine sulfate that are ordinally used for bitterness evaluation and a cinchona extract (an existing additive) obtained from cinchona bark with ethanol or water.

### [Ratio of Combination with Bitterness Component]

The ratio of combination with a bitterness component varies depending on the strength of bitterness to be reduced. The combination ratio can be appropriately adjusted according to bitterness suppression effect. With respect to 1 part by weight of a component having bitterness, there is added 1.25 to 100 parts by dry weight of a stone fruit juice wherein a low molecular weight saccharide has been removed, and preferably, there can be added 5.0 to 50 parts by dry weight of a stone fruit juice wherein a low molecular weight saccharide has been removed.

### [Food or Beverage Products and Pharmaceutical Products, and Production thereof]

A stone fruit juice wherein a low molecular weight saccharide has been removed can be used to add to food or beverage products having bitterness. The added juice, wherein a low molecular weigh saccharide has been removed, is advantageous in that it does not damage original tastes of the food or beverage products and is a low calorie juice. There is no specific limitation on the food or beverage products to which there can be added a stone fruit juice wherein a low molecular weight saccharide has been removed. For example, there may be mentioned beverages to which bitterness components such as isohumulone are added (which are tea-based drinks, fruit drinks, carbonated drinks, vegetable drinks, isotonic drinks, lactic drinks, alcohol drinks and the like) and foods to which bitterness components such as isohumulone are added (which are supplements, jelly, chewing gums, candies, chocolates, ice cream, snacks, and dairy products). The bitterness suppressant of the present invention has functional effect even after being heated and can be used even in processed foods that require heat sterilization. Accordingly, the bitterness suppressant of the present invention can be extremely widely used.

Food or beverage products obtained by adding a stone fruit juice wherein a low molecular weigh saccharide has been removed can be produced by an ordinal method and there can be employed an addition method suitable to a method for producing each food or beverage product. For example, after a stone fruit juice wherein a low molecular weigh saccharide has been removed and a bitterness component are mixed together at high concentration, the mixture is added to various food or beverage products. This method increases bitterness suppression effect. The pH of foods or beverages to which the mixture is added is desirably adjusted to acid (a pH of approximately 3 to 6) in terms of the strength of the effect, although the pH is not limited to this range. Additionally, to food or beverage products having reduced bitterness, there may be added a sweetener, a flavoring material, a preservative, minerals, vitamins, physiologically functional substances, and the like in combination with each other as needed.

A stone fruit juice wherein a low molecular weight saccharide has been removed can be used to add to pharmaceutical products having bitterness. There is no limitation on the form of a pharmaceutical product to which there can be added a stone fruit juice wherein a low molecular weight saccharide has been removed. Examples of such a pharmaceutical product include orally administered drug products, and for example, there can be employed arbitrary forms of drugs, such as capsules, granules, tablets, troches, and syrups. In addition, upon the preparation of a drug, additives conventionally used for preparation, such as a vehicle, may be combined together. A method for producing a pharmaceutical formulation and preparation additives as raw material of the pharmaceutical product are known to those skilled in the art. By, for example, mixing a stone fruit juice wherein a low molecular weight saccharide has been removed with an active ingredient and other preparation additives, there can be produced a pharmaceutical product according to an ordinal method.

The stone fruit juice wherein a low molecular weight saccharide has been removed can be used in an arbitrary form, such as liquid, paste, or powder.

According to a specific aspect of the present invention, there is provided a bitterness suppressant consisting of a stone fruit juice wherein a low molecular weight saccharide has been removed through a dialysis membrane or an ultrafiltration membrane having a fractional molecular weight of 4,000 to 10,000.

According to a specific aspect of the present invention, there is also provided a method for producing a food or beverage product wherein bitterness is suppressed, the method comprising adding to a food or beverage product having bitterness a stone fruit juice wherein a low molecular weight saccharide has been removed through a dialysis membrane or an ultrafiltration membrane having a fractional molecular amount of 4,000 to 10,000.

According to a specific aspect of the present invention, furthermore, there is provided a method for producing a pharmaceutical product wherein bitterness has been masked, the method comprising adding to a pharmaceutical product a stone fruit juice wherein a low molecular weight saccharide has been removed through a dialysis membrane or an ultrafiltration membrane having a fractional molecular amount of 4,000 to 10,000.

According to a specific aspect of the present invention, furthermore, there is provided a method for producing a food or beverage product or a pharmaceutical product wherein bitterness is suppressed, the method comprsing adding to a food or beverage product or a pharmaceutical product a stone fruit juice wherein a low molecular weight saccharide has been removed. Additionally, according to another specific aspect of the invention, there is provided a method for suppressing bitterness in a pharmaceutical product or a food or beverage product, the method comprising adding to a pharmaceutical product or a food or beverage product a stone fruit juice wherein a low molecular weight saccharide has been removed.

According to a specific aspect of the present invention, there is also provided use of a stone fruit juice wherein a low molecular weight saccharide has been removed, in production of a bitterness suppressant. According to a more specific form of the invention, a removal ratio of the low molecular weight saccharide with respect to a sugar content of the juice is no less than 70%. According to a specific form of the invention, also, the low molecular weight saccharide is removed by ultrafiltration or dialysis of the stone fruit juice. According to a more specific form of the invention, also, the stone fruit juice has a molecular weight of preferably 4,000 or more. According to a specific form of the invention, also, the bitterness suppressant is provided in a form of composition. According to a more specific form of the invention, also, the bitterness suppressant is used to add to a food or beverage product or a pharmaceutical product. According to a specific form of the invention, also, the stone fruit is a fruit of the genus Prunus of the rose family. According to a more specific form of the invention, also, the stone fruit is peach, nectarine, plum, cherry fruit, apricot, Japanese apricot, or cherry.

### EXAMPLES

The present invention will be described in detail based on the following Examples but is not limited to those Examples. The notation of concentration (%) in the Examples means w/w% unless otherwise specified.

### Example 1: Preparation Examples of Various Desugared Juices and Sugar Removal Ratios

A 20% solution of 5-fold concentrated peach clear juice (Japan Fruit Processing Company Limited) was prepared and placed in a cellulose dialysis membrane (a fractional molecular weight of 14,000, manufactured by Sanko Junyaku Co., Ltd.) to carry out dialysis overnight in pure water. The dialyzed product was concentrated by an evaporator such that the product had the same volume as that of the initial 20% solution. Regarding the 20% solution and the dialyzed product, Brix measurement was carried out using a saccharimeter (manufactured by ATAGO Co., Ltd.). Table 1 shows the results.

**[Table 1]**

| | 20% juice solution | Desugared juice | Removal ratio |
|---|---|---|---|
| Sugar level (°P) | 8.3 | 0.4 | 95.2% |

It was found that the dialysis reduced a total sugar content by approximately 95%.

### Example 2: Preparation Example of Desugared Juice by Ultrafiltration Process

1 L of a10% solution of 5-fold concentrated peach clear juice (Japan Fruit Processing Company Limited) was prepared and subjected to ultrafiltration using a UF membrane (a fractional molecular weight of 4,000, manufactured by Asahi Kasei Corporation). In the ultrafiltration, 500 mL of water was added to a 2-fold concentrated juice continuously three times to obtain 1 L of a desugared juice. Regarding the 10% solution of 5-fold concentrated peach clear juice and the desugared juice, Brix measurement was carried out by the saccharimeter as in Example 1. Table 2 shows the results. The removal ratio of sugars by the ultrafiltration was 70% and sweetness was hardly felt.

**[Table 2]**

| | 20% juice solution | Desugared juice | Removal ratio |
|---|---|---|---|
| Sugar level (° P) | 3.7 | 0.8 | 78.4% |

### Examples 3 and 4

A concentrated juice of fresh prune plum (Japan Fruit Processing Company Limited) was processed in the completely same way as in Examples 1 and 2 to obtain desugared juices of Examples 3 and 4, respectively. The sugar removal ratio was 93.8% in Example 3 and 87.3% in Example 4.

### Example 5: Preparation Example of Puree, By-Product

A 50% solution of a peach puree (Japan Fruit Processing Company Limited) was prepared and Sumizyme SPC (Aspergillus-derived pectinase manufactured by Shin Nihon Chemical Industry, Co. Ltd.) was added up to 0.1% and allowed to react overnight at 60°C. Enzyme inactivation of the enzyme reaction product was carried out at 80°C for 30 minutes, and then the product cooled down was subjected to solid-liquid separation by centrifugal force. The obtained liquid was dialyzed in the same manner as in Example 1 to obtain a desugared composition. The desugared composition had no sweetness and exhibited suppression effect against a bitterness substance, as in the fruit juice-derived desugared composition.

### Evaluation Example 1

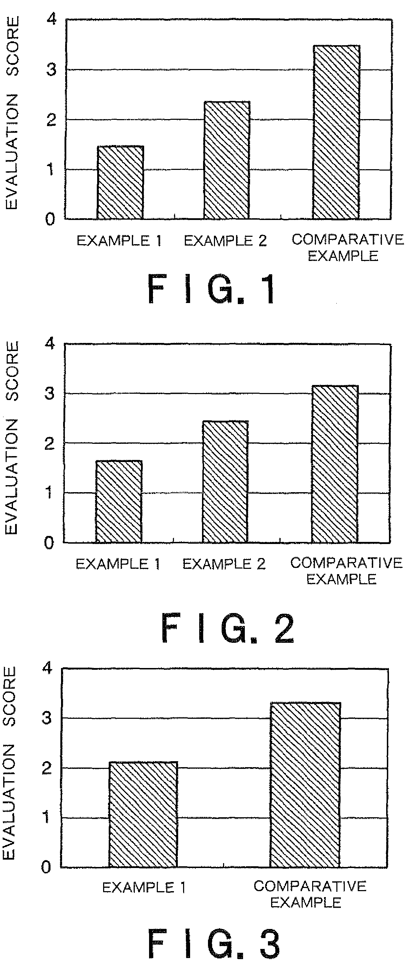

The bitterness substance to be used was prepared by diluting an isomerized hop extract Iso-Extract-30% (manufactured by Hopsteiner Inc.) as isohumulone. Solutions were prepared such that, at an isohumulone concentration of 0.004%, the desugared juice was 0.075% (a preparation product of Example 1) and 0.0375% (a preparation product of Example 2), respectively in terms of dry matter, whereby sensory evaluation was carried out. Additionally, as a control (Comparison Example product 1), there was used a product to which the desugared juice was not added. The strength of bitterness of each solution obtained was evaluated as in Table 3. Results were obtained as mean values of evaluation scores of nine panelists. Fig. 1 shows the results.

**[Table 3]**

| Evaluation item | Evaluation criteria | Evaluation score |
|---|---|---|
| Bitterness | No bitterness is felt. | 0 pt. |
| | Bitterness is slightly felt. | 1 pt. |
| | Bitterness is felt. | 2 pt. |
| | Bitterness is significantly felt. | 3 pt. |
| | Bitterness is so strongly felt that the solution cannot be drunk. | 4 pt. |

Bitterness of the Example products was significantly reduced. Particularly, regarding the preparation product of Example 1, bitterness was reduced down to a slightly bitter level.

### Evaluation Example 2

Evaluation of bitterness reduction effect was also carried out on the preparation products of Examples 3 and 4 in the same manner as in Examples 1 and 2. The evaluation was carried out on a product wherein the desugared juice prepared in Example 3 was added by 0.075% in terms of dry matter, a product wherein the desugared juice prepared in Example 4 was added by 0.0375% in terms of dry matter, and a product without the juice added. Results were obtained as mean values of 10 panelists. Fig. 2 shows the results.

In the desugared juice from the prune fruit juice also, bitterness reduction effect was observed as in peach.

### Evaluation Example 3

By changing the bitterness substance to quinine hydrochloride (Wako Pure Chemical Industries, Ltd.), the same evaluation was carried out. With 0.1 mM (40 ppm) of quinine hydrochloride, there was mixed the desugared juice prepared in Example 1 such that the juice was 0.075% in terms of dry matter to prepare a liquid agent, whereby sensory evaluation was carried out. Additionally, a liquid agent without the desugared juice added was used as a control. Results were obtained as mean values of 10 panelists. Fig. 3 shows the results.

It was also found that there was significant bitterness reduction effect in the liquid agent of quinine hydrochloride.

### Example 6: Production Example of a Food or Beverage Product (Coffee Drink)

Roasted coffee beans were crushed and extracted with hot water of 95°C to obtain a coffee extraction liquid. With respect to 1 part by weight, in terms of isohumulone, of the isomerized hop extract Iso-Extract-30% (manufactured by Hopsteiner Inc.) as an isohumulone, there was mixed 18.75 parts by weight, in terms of dry matter, of a desugared juice of peach prepared in the completely same manner as in Example 1 to prepare a bitterness reduction composition. The bitterness reduction composition was added to the coffee bean extraction liquid up to an isohumulone concentration of 0.008%. Using the coffee bean extraction liquid, a pH adjusting agent was added and prepared. A predetermined amount of the prepared mixture was filled in a tightly sealed container (can) and then, was subjected to sterilization treatment at a temperature of 121°C for 5 minutes. The hop extract-containing coffee drink obtained thus had a reduced isohumulone bitterness and an improved taste quality, as compared to the product without the desugared juice added.

### Example 7: Production Example of a Food or Beverage Product (Jelly)

Into 25 ml of hot water was dissolved 5 g of powdered gelatin and was added 275 ml of grape fruit juice heated to 40°C to stir the mixture. Additionally, granulated sugar was added and 100 g of the mixture was flown into each container. The bitterness reduction composition or the isomerized hop extract used in Example 6 was each added up to a isohumulone concentration of 0.008% and stirred the mixtures, followed by cooling in a refrigerator to produce jellies. In the jelly with the bitterness reduction composition added, bitterness was reduced and taste quality was improved as compared to the jelly containing only the isomerized hop extract.

## Claims

1. A food or beverage product or a pharmaceutical product comprising:
a bitterness suppressant consisting of a juice of a stone fruit wherein a low molecular weight saccharide having a degree of polymerization of from 1 to 20 has been removed, and
a bitterness component selected from humulones and quinine,
wherein 1.25 to 100 parts by dry weight of the juice of a stone fruit wherein a low molecular weight saccharide having a degree of polymerization of from 1 to 20 has been removed is added with respect to 1 part by weight of the bitterness component.

2. The food or beverage product and or a pharmaceutical product according to claim 1, wherein a removal ratio of the low molecular weight saccharide with respect to a sugar content of the juice is no less than 70% by measurement of Brix using a saccharimeter.

3. A method for producing a food or beverage product or a pharmaceutical product wherein bitterness is suppressed, the method comprising adding a juice of a stone fruit wherein a low molecular weight saccharide having a degree of polymerization of from 1 to 20 has been removed to a food or beverage product or a pharmaceutical product which comprises a bitterness component selected from humulones and quinine,
wherein 1.25 to 100 parts by dry weight of the juice of a stone fruit wherein a low molecular weight saccharide having a degree of polymerization of from 1 to 20 has been removed is added with respect to 1 part by weight of the bitterness component.

4. Use of a juice of a stone fruit wherein a low molecular weight saccharide having a degree of polymerization of from 1 to 20 has been removed for suppressing bitterness of a bitterness component selected from humulones and quinine in a pharmaceutical product or a food or beverage product,
wherein 1.25 to 100 parts by dry weight of the juice of a stone fruit wherein a low molecular weight saccharide having a degree of polymerization of from 1 to 20 has been removed is added with respect to 1 part by weight of the bitterness component.

## Patentansprüche

1. Nahrungsmittel- oder Getränkeprodukt oder pharmazeutisches Produkt, umfassend:
ein Bitterkeitsunterdrückungsmittel, bestehend aus einem Saft eines Steinobstes, bei dem ein Saccharid mit niedrigem Molekulargewicht, das einen Polymerisationsgrad von 1 bis 20 aufweist, entfernt worden ist, und
eine Bitterkeitskomponente, ausgewählt aus Humulonen und Chinin,
wobei 1,25 bis 100 Trockengewichtsteile des Saftes eines Steinobstes, bei dem ein Saccharid mit niedrigem Molekulargewicht, das einen Polymerisationsgrad von 1 bis 20 aufweist, entfernt worden ist, bezogen auf 1 Gewichtsteil der Bitterkeitskomponente hinzugefügt sind.

2. Nahrungsmittel- oder Getränkeprodukt oder pharmazeutisches Produkt gemäß Anspruch 1, wobei ein Entfernungsverhältnis des Saccharids mit niedrigem Molekulargewicht bezogen auf den Zuckergehalt des Saftes nicht weniger als 70% gemäß Brix-Messung unter Verwendung eines Saccharimeters beträgt.

3. Verfahren zur Herstellung eines Nahrungsmittel- oder Getränkeprodukts oder eines pharmazeutischen Produkts, bei dem Bitterkeit unterdrückt wird, wobei das Verfahren das Hinzufügen eines Saftes eines Steinobstes, bei dem ein Saccharid mit niedrigem Molekulargewicht, das einen Polymerisationsgrad von 1 bis 20 aufweist, entfernt worden ist, zu einem Nahrungs- oder Getränkeprodukt oder einem pharmazeutischen Produkt, umfassend eine Bitterkeitskomponente, ausgewählt aus Humulonen und Chinin, umfasst,
wobei 1,25 bis 100 Trockengewichtsteile des Saftes eines Steinobstes, bei dem ein Saccharid mit niedrigem Molekulargewicht, das einen Polymerisationsgrad von 1 bis 20 aufweist, entfernt worden ist, bezogen auf 1 Gewichtsteil der Bitterkeitskomponente hinzugefügt werden.

4. Verwendung eines Saftes eines Steinobstes, bei dem ein Saccharid mit niedrigem Molekulargewicht, das einen Polymerisationsgrad von 1 bis 20 aufweist, entfernt worden ist, zur Unterdrückung von Bitterkeit einer Bitterkeitskomponente, ausgewählt aus Humulonen und Chinin, in einem pharmazeutischen Produkt oder einem Nahrungsmittel- oder Getränkeprodukt,
wobei 1,25 bis 100 Trockengewichtsteile des Saftes eines Steinobstes, bei dem ein Saccharid mit niedrigem Molekulargewicht, das einen Polymerisationsgrad von 1 bis 20 aufweist, entfernt worden ist, bezogen auf 1 Gewichtsteil der Bitterkeitskomponente hinzugefügt werden.

## Revendications

1. Produit alimentaire ou à boire ou produit pharmaceutique comprenant :
un agent de suppression d'amertume consistant en un jus d'un fruit à noyau dans lequel un saccharide de faible masse moléculaire ayant un degré de polymérisation de 1 à 20 a été éliminé, et
un composant d'amertume sélectionné parmi les humulones et la quinine,
dans lequel 1,25 à 100 parties en poids sec du jus d'un fruit à noyau dans lequel un saccharide de faible masse moléculaire ayant un degré de polymérisation de 1 à 20 a été éliminé sont ajoutées par rapport à 1 partie en poids du composant d'amertume.

2. Produit alimentaire ou à boire et/ou produit pharmaceutique selon la revendication 1, dans lequel un rapport d'enlèvement du saccharide de faible masse moléculaire par rapport à une teneur en sucre du jus n'est pas inférieure à 70 % par mesure de Brix en utilisant un saccharimètre.

3. Méthode de production d'un produit alimentaire ou à boire ou d'un produit pharmaceutique dans laquelle l'amertume est supprimée, la méthode comprenant l'ajout d'un jus d'un fruit à noyau dans lequel un saccharide de faible masse moléculaire ayant un degré de polymérisation de 1 à 20 a été éliminé à un produit alimentaire ou à boire ou à un produit pharmaceutique qui comprend un composant d'amertume sélectionné parmi les humulones et la quinine,
dans laquelle 1,25 à 100 parties en poids sec du jus d'un fruit à noyau dans lequel un saccharide de faible masse moléculaire ayant un degré de polymérisation de 1 à 20 a été éliminé sont ajoutées par rapport à 1 partie en poids du composant d'amertume.

4. Utilisation d'un jus d'un fruit à noyau dans lequel un saccharide de faible masse moléculaire ayant un degré de polymérisation de 1 à 20 a été éliminé pour supprimer l'amertume d'un composant d'amertume sélectionné parmi les humulones et la quinine dans un produit pharmaceutique ou un produit alimentaire ou à boire,
dans laquelle 1,25 à 100 parties en poids sec du jus d'un fruit à noyau dans lequel un saccharide de faible masse moléculaire ayant un degré de polymérisation de 1 à 20 a été éliminé sont ajoutées par rapport à 1 partie en poids du composant d'amertume.
